# EUROPÄISCHE PATENTSCHRIFT

(11) **EP 0 423 532 B1**
(45) Veröffentlichungstag und Bekanntmachung des Hinweises auf die Patenterteilung: **09.03.1994**
(21) Anmeldenummer: 90118808.6
(22) Anmeldetag: 01.10.1990
(51) Int. Cl.: C01B 33/34, B01J 29/06

(54) **Verfahren zur Herstellung synthetischer feinkristalliner Metallsilikate**
Method for the preparation of synthetic fine crystalline metal silicates
Procédé de préparation de silicates de métaux finement cristallisés synthétiques

(30) Priorität: 16.10.1989 DD 333612
(43) Veröffentlichungstag der Anmeldung: 24.04.1991
(73) Patentinhaber: VAW Aluminium AG, D-53117 Bonn (DE); LEUNA-WERKE AG, D-06236 Leuna (DE)
(72) Erfinder: Becker, Karl, Dr., O-4807 Bad Kösen (DE); Unger, Baldur, Dr., O-6421 Reichmannsdorf (DE); Thome, Roland, Dr., W-5300 Bonn 1 (DE); Tissler, Arno, Dr., W-5300 Bonn 1 (DE); Schwieger, Wilhelm, Dr., O-04020 Halle (DE); Tschritter, Hartmut, Dr., O-06500 Gera (DE)
(74) Vertreter: Müller-Wolff, Thomas, Dipl.-Ing.

(56) Entgegenhaltungen:
- EP-A- 0 111 147
- DE-A- 2 017 807
- US-A- 4 211 760
- CHEMICAL ABSTRACTS, vol. 86, no. 18, 2. Mai 1977, Columbus, Ohio, US;abstract no. 132607P, WOLF & AL.: 'HYDROTHERMAL SYNTHESIS OF TYPE A ZEOLITE IN THE PRESENCE OF VANADIUM SALTS' Seite 771; Spalte L; & Z. CHEM. 1976, 16(12) 498-499

## Beschreibung

Die Erfindung betrifft ein Verfahren zur Herstellung synthetischer feinkristalliner Metallsilikate der Zusammensetzung 0,8 bis 1,5 Na₂O x (Al₂O₃ + xM₂O₃) x 10 bis 100 SiO₂ mit x = 0,05 bis 5 und M für ein oder mehrere Elemente der 3. bis 7. Nebengruppe des PSE, und Pentasil-typischem Röntgendiffraktogramm sowie die Verwendung der daraus hergestellten synthetischen kristallinen Metallsilikate zur Herstellung von Katalysatoren.

Aufgrund ihrer spezifischen strukturellen und oberflächenchemischen Eigenschaften erweisen sich kristalline alumosilikatische Molekularsiebe als für katalytische und Adsorptionszwecke außerordentlich geeignete Substanzen. So ist aus der DE-A-2 017 807 ein Verfahren zur Herstellung eines Natriummetallsilikats bekannt, wobei das Molverhältnis SiO₂/Al₂O₃ zwischen 2,5 und 10 liegt. Die hierdurch hergestellten Zeolithe weisen eine Faujasit-Struktur auf, wobei während der Entstehung des Molekularsiebes Metallionen der Gruppen I, II, III, IV, V, VI, VII oder VIII des Periodensystems einschließlich der Edelmetalle eingebaut werden. Dabei wird eine Lösung des Metallsalzes zu einer Lösung von Natriumsilikat oder Natriumaluminat gegeben, wobei ein Komplexierungsmittel wie Natriumtartrat oder ein anderes vergleichbares Komplexierungsmittel dem Gemisch zugesetzt wird.

Nach EP-A-111 147 werden titan-, zirkon- und/oder hafniumhaltige Zeolithe hergestellt, die folgende Strukturen aufweisen:
Zeolith T oder Zeolith ZSM-34.
Vorzugsweise werden dabei Impfkristalle eines Zeoliths ähnlichen Strukturtyps zugesetzt. Das Syntheseverfahren ist sehr langwierig und kann bis zu 2000 Stunden dauern.

In US-A-4 211 760 wird ein Verfahren zur Herstellung eines Zeoliths mit dem Namen "Upsilon" beschrieben, der sich aufgrund seines geringen Porenradius (3,4 Å) insbesondere für die Trocknung von Gasströmen z.B. wasserdampfhaltigen Strömen eignet. Zur Herstellung dieses Zeolithtyps wird Cr₂O₃ zugesetzt, wobei das zugesetzte Cr₂O₂ offenbar "struktur dirigierend" in Richtung der Bildung des Zeoliths "Upsilon" wirkt.

Neue Entwicklungen auf diesem Gebiet umfassen die Zeolithe vom sogenannten Pentasiltyp, als deren Hauptvertreter der ZSM-5 und der ZSM-11 bekannt geworden sind (P.A. Jacobs u. J.A. Martens, "Synthesis of High-Silica Alumosilicate Zeolites", Elsevier, Amsterdam, 1987). Zeolithe dieses Typs zeichnen sich durch eine hohe Stabilität gegenüber der Einwirkung von Mineralsäuren, Wasserdampf und hohen Temperaturen aus und können deshalb für eine ganze Reihe, insbesondere formselektiver Umwandlungen als Katalysator bzw. Katalysatorkomponente eingesetzt werden. Zu solchen Reaktionen zählen bekanntermaßen die Umwandlung von Methanol zu Kohlenwasserstoffen im VK-Siedebereich bzw. zu Alkenen (US-A-3.333.250), die selektive Umsetzung von n-Paraffinen in technischen Kohlenwasserstoffgemischen in Hinblick auf die Verbesserung des Kälteverhaltens von DK-Fraktionen (DD-A-160.385), die paraselektive Umwandlung oder Herstellung von Alkylaromaten (US-A-4.097.543, US-A-4.052.476) oder auch die Umsetzung von Alkoholen oder Ethern zu Aminen (US-A-4.082.805).

Bekanntlich werden die in katalytischen Reaktionen wesentlichen Parameter zeolithhaltiger Katalysatoren, wie Aktivität, Selektivität und Langzeitstabilität insbesondere von der Zusammensetzung der Katalysatorkomponente, speziell vom Gitteraluminiumgehalt und von der Art und der Menge im Zeolith enthaltener Kationen bestimmt. Wie bereits erwähnt, sind Pentasilzeolithe aufgrund der ihnen eigenen Variabilität bezüglich ihrer Zusammensetzung für eine zweckgerichtete Herstellung katalytischer Aktivkomponenten, teils durch Direktsynthese, teils durch Nachbehandlung, besonders geeignet.
Neben den genannten Einflußgrößen ist es aber insbesondere die Morphologie, speziell die Größe und die Homogenität des eingesetzten Zeolithkristallisats, die die katalytischen Parameter derartiger Systeme, besonders deren Langzeitstabilität in wesentlichem Maße derart mitbestimmen, daß feinteilige Kristallisate ein verbessertes Langzeitverhalten zeigen (M. Sugimot et al., Zeolites 7(1987)503). Zur Herstellung von feinteiligem Pentasilzeolithkristallisat sind verschiedene Verfahren beschrieben. Dazu zählen u.a.: die Einhaltung eines bestimmten pH-Wertes und Temperaturregimes während der Synthese (z. B. EP-A-186.480), Veränderungen in der Art und Menge an zugesetzten Kristallkeimen (z. B. EP-A-173.901), Zusatz von NaCl zur Synthesemischung (Z. Zhao, Hunan Shifan Daxue Ziran Kexue Xuebao 10(1987)47), Veränderungen der Menge an freier Template-Verbindung (V.M. Romannikov et al., Zeolites 3(1983)311) sowie unterschiedliche Verfahren einer thermischen und mechanischen Nachbehandlung.

Nachteilig ist bei allen beschriebenen Verfahren, daß die Zeolithsynthese stets in Anwesenheit einer als sogenanntes Template wirkenden Verbindung durchgeführt wird, bzw. zur Nachbehandlung in Gegenwart organischer Template-Verbindungen hergestellte Zeolithe eingesetzt werden. Auf diese Art und Weise hergestellte Zeolithe erfordern einen hohen technischen und ökonomischen Aufwand, der sowohl die Kosten für die Bereitstellung des meist verhältnismäßig teueren Templates als auch die Rückgewinnung der unumgesetzten Template-Verbindung aus der Syntheseablauge umfaßt. Weiterhin nachteilig wirkt sich aus, daß die Anwesenheit organischer Verbindungen synthetisierten Zeolithe einen Teil dieser Substanzen im Zeolithgefüge eingebaut enthalten. Die notwendige Entfernung dieser Stoffe erfolgt generell durch Abbrennen bei höheren Temperaturen, einem Vorgang, der zum einen mit erhöhtem Energieaufwand verbunden ist, zum anderen erhöhte Aufwendungen zur Entsorgung der toxikologisch meist nicht unbedenklichen Abgase erfordert und letztlich oft unvermeidlich zu einer durch thermische Belastungen hervorgerufenen partiellen Schädigung des Zeolithen führt.

Der Erfindung liegt die Aufgabe zugrunde, ein Verfahren zur Herstellung synthetischer feinkristalliner Metallsilikate zu schaffen, welches kostengünstig und umweltfreundlich betrieben werden kann.

Aufgabe der Erfindung ist es, ein Verfahren zu schaffen, welches die Herstellung von Metallsilikaten mit Zeolithgefüge, bestehend aus einem Haufwerk kleiner Kristallite einheitlicher Morphologie, gestattet.

Die Aufgabe wird erfindungsgemäß durch die im Hauptanspruch angegebenen Merkmale gelöst. Weitere Ausgestaltungen der Erfindung sind den Unteransprüchen zu entnehmen.

Überraschend war, daß nur durch Zusatz von Verbindungen der genannten Nebengruppenelemente im angegebenen Konzentrationsbereich, jedoch ohne Zusatz einer organischen oder Stickstoffverbindung, ein feinteiliges und morphologisch einheitliches Kristallisat mit Kristallitgrößen im wesentlichen unter 1 µm entsteht.
Als Quellen der zur Synthese verwendeten Elemente bzw. deren Oxide können beispielsweise dienen:
- für Natriumoxid: Natriumhydroxid oder Natriumnitrat;
- für Siliziumdioxid: pyrogene oder Fällungskieselsäuren, Kieselsäuresole, Alkalimetallsilikate und/oder Wasserglaslösungen;
- für die genannten Nebengruppenelemente: Salze (insb. Nitrate), hydratisierte bzw. hydratisierbare Oxide;
- für Aluminiumoxid: hydratisiertes oder hydratisierbares Aluminiumoxid, Aluminiumsalze, insb. Aluminiumnitrat und/oder Aluminatlösungen.

Die Vermischung der Ausgangsstoffe kann diskontinuierlich in den für die Reaktion vorgesehenen Gefäßen, jedoch auch kontinuierlich in einer der Kristallisation vorgelagerten Stufe erfolgen. Typische Zusammensetzungen der Reaktionsmischung, ausgedrückt in Molverhältnissen der eingesetzten Elemente bzw. deren Oxide sind:

| | |
|---|---|
| Na₂O/SiO₂ | 0,05 bis 0,5 |
| H₂O/SiO₂ | 5 bis 150 |
| M/Si | 0,001 bis 0,2 |
| SiO₂/Al2O₃ | 10 bis 10 000. |

Bei einer Reaktionstemperatur von 403 K bis 593 K wird diese Mischung unter hydrothermalen Bedingungen bis zur Kristallisation gehalten, das Kristallisat anschließend von der Mutterlauge getrennt, gewaschen und bei 383 K getrocknet. Durch 1 bis 24 stündiges Altern der Reaktionsmischung bei Raumtemperatur und/oder durch Zugabe von Kristallkeimen kann die Kristallisation beschleunigt und die Einheitlichkeit des Kristallisats verbessert werden. Die auf diese Weise erhaltenen Metallsilikate erzeugen charakteristische Röntgenbeugungslinien, sind frei von organischen und Stickstoffverbindungen und weisen im dehydratisierten Zustand folgenden Zusammensetzung auf:
0,8 bis 1,5 Na₂O * (Al₂O₃ + x M₂O₃) * 10 bis 100 SiO₂ mit x = 0,05 bis 5.

Die erfindungsgemäßen Metallsilikate können für den Einsatz z. B. in Katalysatoren durch auf dem Fachgebiet bekannte Techniken modifiziert und/oder geformt werden.

### Ausführungsbeispiele

Es bedeuten Ma.-% Masseanteile in Prozent.

### Beispiel 1 (Vergleichsbeispiel)

In Anlehnung an DD 207.186 wird eine Lösung hergestellt aus 2,21 g Natriumhydroxid (p.a.), 243 g Wasser und 8,46 g Natriumaluminat (ca. 13 Ma.-% Al₂O₃). Zu dieser alkalischen Lösung werden bei Raumtemperatur unter intensivem Rühren 86,05 g Natriumstabilisiertes Kieselsol (ca. 31 Ma.-% SiO₂) zugefügt und homogenisiert.
Die Zusammensetzung der Reaktionsmischung ergibt sich zu folgenden Molverhältnissen:

| | |
|---|---|
| Na₂O/SiO₂ | 0,1 |
| H₂O/Na₂O | 300 |
| SiO₂/Al₂O₃ | 40. |

Das Reaktionsgemisch wird unter Eigendruck bei 473 K zur Kristallisation gebracht. Nach einer Kristallisationszeit von 9 Stunden wird ein Produkt erhalten, das Pentasilstruktur besitzt und die in Tabelle 1 angeführten typischen Röntgenbeugungslinien (hier ausgedrückt in Werten der entsprechenden Netzebenenabstände) hervorruft. Die auf beschriebene Weise hergestellte Probe besitzt Vergleichscharakter.

**Tabelle 1**

| **Werte der charakteristischen Röntgenbeugungslinien der Vergleichsprobe entsprechend Beispiel 1** | |
|---|---|
| Netzebenenabstand (nm) | relative Intensität |
| 1,11 +/- 0,02 | st |
| 0,980 +/- 0,02 | st |
| 0,587 +/- 0,01 | s |
| 0,557 +/- 0,01 | s |
| 0,548 +/- 0,01 | ss |
| 0,421 +/- 0,01 | s |
| 0,386 +/- 0,007 | sst |
| 0,381 +/- 0,007 | st |
| 0,372 +/- 0,005 | m |
| 0,369 +/- 0,005 | m |
| 0,362 +/- 0,01 | m |
| 0,340 +/- 0,01 | s |
| 0,329 +/- 0,003 | ss |
| 0,302 +/- 0,01 | s |
| 0,297 +/- 0,01 | s |
| 0,294 +/- 0,01 | s |

### Beispiel 2

Es wird eine Lösung, bestehend aus 3,58 g Natriumhydroxid, 4,56 g Cr(NO₃)₃*9 H₂O, 243 g Wasser und 8,46 g Natriumaluminat bereitet, unter kräftigem Rühren 86,05 g Natrium-stabilisiertes Kieselsol zugesetzt und das Reaktionsgemisch intensiv homogenisiert. Die molare Zusammensetzung der Reaktionspartner ergibt sich zu:

| | |
|---|---|
| Na₂O/SiO₂ | 0,16 |
| Cr₂O₃/Al₂O₃ | 1 |
| H₂O/Na₂O | 185 |
| SiO₂/Al₂O₃ | 40. |

Das Reaktionsgemisch wird unter Eigendruck bei 473 K zur Kristallisation gebracht. Nach 16 Stunden wird ein Produkt erhalten, das ein Pentasil-typisches Röntgendiffraktogramm hervorruft und sich durch Feinteiligkeit und Homogenität des Kristallisats auszeichnet.

### Beispiel 3

Es wird eine Lösung, bestehend aus 4,95 g Natriumhydroxid, 9,12 g Cr(NO₃)₃*9 H₂O, 243 g Wasser und 8,46 g Natriumaluminat bereitet, unter kräftigem Rühren 86,05 g Natrium-stabilisiertes Kieselsol zugesetzt und das Reaktionsgemisch intensiv homogenisiert. Die molare Zusammensetzung der Reaktionspartner ergibt sich zu:

| | |
|---|---|
| Na₂O/SiO₂ | 0,22 |
| H₂O/Na₂O | 134 |
| Cr/Al | 1 |
| SiO₂/Al₂O₃ | 40. |

Das Reaktionsgemisch wird unter Eigendruck bei 458 K zur Kristallisation gebracht. Nach 26 Stunden wird ein Produkt erhalten, das ein Pentasil-typisches Röntgendiffraktogramm hervorruft. Es besteht aus Kristalliten mit einer einheitlichen Größe von weniger als 0.5 µm.

### Beispiel 4

Es wird eine Lösung hergestellt aus 3,12 g Natriumhydroxid, 3,27 g Mn(NO₃)₃*6 H₂O, 243 g Wasser, 8,46 g Natriumaluminat, in diese Lösung 86,05 g Natrium-stabilisiertes Kieselsol eingebracht und intensiv homogenisiert. Die molare Zusammensetzung der Reaktionsmischung dergibt sich zu:

| | |
|---|---|
| Na₂O/SiO₂ | 0,14 |
| H₂O/Na₂O | 212 |
| Mn/Al | 1 |
| SiO₂/Al₂O₃ | 40. |

Das Reaktionsgemisch wird unter Eigendruck bei 473 K zur Kristallisation gebracht und nach 12 Stunden ein Produkt abgetrennt, das sich röntgenographisch als Pentasil-typisches Kristallisat erweist und eine einheitliche Morphologie besitzt.

### Beispiel 5

Jeweils 50 g der gemäß Beispiel 1 bis 4 hergestellten Produkte wurden feingemahlen und zweimal für jeweils 3 Stunden in 3 l einer 0,5 n Ammoniumnitratlösung bei 363 K gerührt, filtriert und nitratfrei gewaschen. Der Filterkuchen wurde einem speziell vorbereiteten Böhmithydrosol zugemischt und auf bekannte Weise zu Tropfkugeln geformt. Das Massenverhältnis der Feststoffgehalte von Zeolithkomponente zu Aluminiumoxid betrug 70 zu 30. Die Tropfkugeln wurden anschließend 3 Stunden bei 773 K getempert.
30 g dieser Katalysatorformlinge wurden bei 593 K, einem Druck von 3 MPa und einer Belastung von 3 g/gh in einem Technikumsreaktor zur Umwandlung von Methanol zu vorwiegend flüssigen Kohlenwasserstoffen eingesetzt. Als Maß für die Standzeit der Katalysatorproben diente die Reaktionsdauer bis zum Durchbruch des Methanols durch die Katalysatorschüttung.
Die Ergebnisse der Testreaktion sind in Tabelle 2 dargestelllt:

**Tabelle 2**

| Katalysator, hergestellt aus Zeolithproben gemäß Beispiel | Standzeit, h |
|---|---|
| 1 | 45 |
| 2 | 70 |
| 3 | 75 |
| 4 | 68. |

## Patentansprüche

1. Verfahren zur Herstellung synthetischer feinkristalliner Metallsilikate der Zusammensetzung 0,8 bis 1,5 Na₂O x (Al₂O₃ + xM₂O₃) x 10 bis 100 SiO₂ mit x = 0,05 bis 5 und M für ein oder mehrere Elemente der 3. bis 7. Nebengruppe des PSE, und Pentasil-typischem Röntgendiffraktogramm,
dadurch gekennzeichnet,
daß eine Reaktionsmischung, die Verbindungen von Silicium, Natrium und mindestens einem Element der 3. bis 7. Nebengruppe des PSE, insbesondere Chrom, Molybdän, Wolfram oder Mangan, neben Hydroxidionenn und Wasser sowie mindestens eine Aluminiumverbindung in den Molverhältnissen
Na₂O/SiO₂ von 0,05 bis 0,5
H₂O/SiO₂ von 5 bis 150
M/Si von 0,001 bis 0,2
SiO₂/Al₂O₃ von 10 bis 10 000,
jedoch keine organische oder Stickstoffverbindung enthält, einem Kristallisationsprozeß unterworfen wird.

2. Verfahren nach Anspruch 1,
dadurch gekennzeichnet,
daß die Reaktion in einem Zeitraum von 1 bis 100 Stunden unter hydrothermalen Bedingungen bei 403 bis 593 K in einem Kristallisationsprozeß durchgeführt wird.

3. Ein Verfahren gemäß Anspruch 1, in dem SiO₂/Al₂O₃ im Bereich von 30 bis 60 liegt.

4. Ein Verfahren gemäß Anspruch 1, in dem M/Si im Bereich von 0,02 bis 0,15 liegt.

5. Ein Verfahren gemäß Anspruch 1, in dem Na₂O/SiO₂ im Bereich von 0,08 bis 0,2 liegt.

6. Ein Verfahren gemäß Anspruch 1, in dem H₂O/SiO₂ im Bereich von 20 bis 100 liegt.

7. Ein Verfahren gemäß Anspruch 2, welches über einen Zeitraum von 8 bis 40 Stunden betrieben wird.

8. Verfahren gemäß Anspruch 2, welches bei einer Temperatur von 423 K bis 533 K betrieben wird.

9. Ein Verfahren gemäß den Ansprüchen 1 bis 8, bei dem die Reaktionsmischung vor der Kristallisation über einen Zeitraum von 1 bis 24 Stunden bei Raumtemperatur gealtert wird.

10. Ein Verfahren gemäß den Ansprüchen 1 bis 9, bei dem der Reaktionsmischung 0,5 bis 10 prozentuale Massenanteile eines synthetischen kristallinen Silikats zugesetzt wird, das zumindest die folgenden Netzebenenabstände besitzt:
| Netzebenenabstand (nm) | relative Intensität |
|---|---|
| 1,11 +/- 0,02 | st |
| 0,980 +/- 0,02 | st |
| 0,587 +/- 0,01 | s |
| 0,557 +/- 0,01 | s |
| 0,548 +/- 0,01 | ss |
| 0,421 +/- 0,01 | s |
| 0,386 +/- 0,007 | sst |
| 0,381 +/- 0,007 | st |
| 0,372 +/- 0,005 | m |
| 0,369 +/- 0,005 | m |
| 0,362 +/- 0,01 | m |
| 0,340 +/- 0,01 | s |
| 0,329 +/- 0,003 | ss |
| 0,302 +/- 0,01 | s |
| 0,297 +/- 0,01 | s |
| 0,294 +/- 0,01 | s |

11. Ein Verfahren gemäß den Ansprüchen 1 bis 10, bei dem ein synthetisches feinkristallines Metallsilikat entsteht, welches die in Anspruch 10 angegebenen charakteristischen Rontgenbeugungslinien hervorruft, frei von organischen und Stickstoffverbindungen ist und im dehydratisierten Zustand folgende Zusammensetzung aufweist:
0,8 bis 1,5 Na₂O * (Al₂O₃ + x M₂O₃) * 10 bis 100 SiO₂ mit x = 0,05 bis 5, wobei M für die unter Anspruch 1 genannten Elemente steht.

12. Verwendung eines gemäß der vorhergehenden Ansprüche hergestellten synthetischen kristallinen Metallsilikats zur Herstellung von Katalysatoren.

13. Verwendung eines Katalysators gemäß Anspruch 12 in katalytischen Verfahren.

## Claims

1. A process for the preparation of synthetic finely crystalline metal silicates with the composition 0.8 to 1.5 Na₂O x (Al₂O₃ + xM₂O₃) x 10 to 100 SiO₂, where x = 0.05 to 5 and M represents one or more elements from the 3rd to 7th B groups of the Periodic Table, and with X-ray diffraction patterns which are typical of pentasil, characterised in that
a reaction mixture, which contains compounds of silicon, sodium and at least one element from the 3rd to 7th B groups of the Periodic Table, especially chromium, molybdenum, tungsten or manganese, as well as hydroxide ions and water and at least one aluminium compound in the molar ratios
Na₂O/SiO₂ of 0.05 to 0.5
H₂O/SiO₂ of 5 to 150
M/Si of 0.001 to 0.2
SiO₂/Al₂O₃ of 10 to 10 000,
but which do not contain any organic or nitrogen compounds, is subjected to a crystallisation process.

2. A process according to claim 1,
characterised in that
the reaction is performed in a crystallisation process over a period of 1 to 100 hours under hydrothermal conditions at 403 to 593 K.

3. A process according to Claim 1, in which the SiO₂/Al₂O₃ ratio is in the range 30 to 60.

4. A process according to claim 1, in which the M/Si ratio is in the range 0.02 to 0.15.

5. A process according to Claim 1, in which the Na₂O/SiO₂ ratio is in the range 0.08 to 0.2.

6. A process according to Claim 1, in which the H₂O/SiO₂ ratio is in the range 20 to 100.

7. A process according to Claim 2, which is operated over a period of 8 to 40 hours.

8. A process according to Claim 2, which is operated at a temperature of 423 to 533 K.

9. A process according to Claims 1 to 8, in which the reaction mixture is aged for a period of 1 to 24 hours at room temperature before crystallisation.

10. A process according to Claims 1 to 9, in which 0.5 to 10 percentage parts by weight of a synthetic crystalline silicate which has at least the following lattice distances, is added to the reaction mixture:
| lattice distances | relative intensity |
|---|---|
| 1.11 +/- 0.02 | strong |
| 0.980 +/- 0.02 | strong |
| 0.587 +/- 0.01 | weak |
| 0.557 +/- 0.01 | weak |
| 0.548 +/- 0.01 | very weak |
| 0.421 +/- 0.01 | weak |
| 0.386 +/- 0.007 | very strong |
| 0.381 +/- 0.007 | strong |
| 0.372 +/- 0.005 | medium |
| 0.369 +/- 0.005 | medium |
| 0.362 +/- 0.01 | medium |
| 0.340 +/- 0.01 | weak |
| 0.329 +/- 0.003 | very weak |
| 0.302 +/- 0.01 | weak |
| 0.297 +/- 0.01 | weak |
| 0.294 +/- 0.01 | weak |

11. A process according to Claims 1 to 10, in which a synthetic finely crystalline metal silicate is produced which gives rise to the characteristic X-ray diffraction lines cited in Claim 10, is free of organic and nitrogen compounds and has the following composition in the dehydrated state: 0.8 to 1.5 Na₂O * (Al₂O₃ + xM₂O₃) * 10 to 100 SiO₂,
where x = 0.05 to 5, whereby M represents the elements mentioned in Claim 1.

12. Use of a synthetic crystalline metal silicate prepared in accordance with the preceding Claims for the preparation of catalysts.

13. Use of a catalyst according to Claim 12 in catalytic processes.

## Revendications

1. Procédé pour la préparation de silicates métalliques synthétiques en cristaux fins de composition 0,8 à 1,5 Na₂O x (Al₂O₃ + xM₂O₃) x 10 à 100 SiO₂, avec x = 0,05 à 5 et M représentant un ou plusieurs éléments du troisième au septième sous-groupes de la Classification Périodique, avec un diffractogramme de rayons X typique des pentasils, caractérisé en ce que l'on soumet à cristallisation un mélange de réaction contenant des composés du silicium, du sodium et d'au moins un élément du troisième au septième sous-groupes de la Classification Périodique, en particulier le chrome, le molybdène, le tungstène ou le manganèse, avec des ions hydroxyde et de l'eau, et au moins un dérivé de l'aluminium, à des rapports molaires
Na₂O/SiO₂ de 0,05 à 0,5
H₂O/SiO₂ de 5 à 150
M/Si de 0,001 à 0,2
SiO₂/Al₂O₃ de 10 à 10.000
mais en l'absence de composés organiques ou de composés de l'azote.

2. Procédé selon la revendication **1**, caractérisé en ce que la composition du mélange de réaction, exprimée par les rapport molaires entre les éléments ou leurs oxydes, correspond à
Na₂O/SiO₂ 0,05 à 0,5
H₂O/SiO₂ 5 à 150
M/Si 0,001 à 0,2
SiO₂/Al₂O₃ 10 à 10.000
M représentant un ou plusieurs éléments du troisième au septième sous-groupe de la Classification Périodique.

3. Procédé selon la revendication **1**, dans lequel le rapport SiO₂/Al₂O₃ va de 30 à 60.

4. Procédé selon la revendication **1**, dans lequel le rapport M/Si se situe dans l'intervalle de 0,02 à 0,15.

5. Procédé selon la revendication **1**, dans lequel le rapport Na₂O/SiO₂ se situe dans l'intervalle de 0,08 à 0,2.

6. Procédé selon la revendication **1**, dans lequel le rapport H₂O/SiO₂ se situe dans l'intervalle de 20 à 100.

7. Procédé selon la revendication **2**, mis en oeuvre en une durée de 8 à 40 heures.

8. Procédé selon la revendication **2**, mis en oeuvre à une température de 423 K à 533 K.

9. Un procédé selon l'une des revendications **1** à **8**, dans lequel le mélange de réaction, avant la cristallisation, est soumis à une maturation de 1 à 24 heures à température ambiante.

10. Un procédé selon l'une des revendications **1** à **9**, dans lequel on ajoute au mélange de réaction de 0,5 à 10 % en poids d'un silicate cristallisé synthétique présentant au moins des distances entre plans réticulaires suivants :
| Distances des plans réticulaires, nm | Intensité relative |
|---|---|
| 1,11 +/- 0,02 | Forte |
| 0,980 +/- 0,02 | Forte |
| 0,587 +/- 0,01 | Faible |
| 0,557 +/- 0,01 | Faible |
| 0,548 +/- 0,01 | Très faible |
| 0,421 +/- 0,01 | Faible |
| 0,386 +/- 0,007 | Très forte |
| 0,381 +/- 0,007 | Forte |
| 0,372 +/- 0,005 | Moyenne |
| 0,369 +/- 0,005 | Moyenne |
| 0,362 +/- 0,01 | Moyenne |
| 0,340 +/- 0,01 | Faible |
| 0,329 +/- 0,003 | Très faible |
| 0,302 +/- 0,01 | Faible |
| 0,297 +/- 0,01 | Faible |
| 0,294 +/- 0,01 | Faible |

11. Un procédé selon les revendications **1** à **10**, dans lequel on forme un silicate métallique synthétique en cristaux fins présentant les raies de diffraction de rayons X caractéristiques indiquées dans la revendication **10**, exempt de composés organiques et de composés azotés et qui, à l'état déshydratés, présente la composition suivante : 0,8 à 1,5 Na₂O x (Al₂O₃ + xM₂O₃) x 10 à 100 SiO₂, avec x = 0,05 à 5 et M représentant les éléments spécifiés dans la revendication **1**.

12. Utilisation d'un silicate métallique synthétique cristallisé préparé comme décrit dans les revendications précédentes pour la préparation de catalyseurs.

13. Utilisation d'un catalyseur selon la revendication **12** dans des opérations catalytiques.
